# EUROPÄISCHE PATENTSCHRIFT

(11) **EP 1 731 105 B1**
(45) Veröffentlichungstag und Bekanntmachung des Hinweises auf die Patenterteilung: **06.05.2009**
(21) Anmeldenummer: 06011738.9
(22) Anmeldetag: 07.06.2006
(51) Int. Cl.: A61B 17/34, A61B 19/00

(54) **Vorrichtung zum Schaffen eines freien transkutanen Zuganges zu einem endoskopischen Operationsgebiet**
Device for establishing free transcutaneous access to an endoscopic surgical area
Dispositif permettant de pratiquer un accès transcutané à une zone d'opération chirurgicale endoscopique

(30) Priorität: 09.06.2005 DE 102005026467
(43) Veröffentlichungstag der Anmeldung: 13.12.2006
(73) Patentinhaber: University of Dundee, Dundee Dundee City DD1 4HN (GB)
(72) Erfinder: Cuschieri, Alfred, Prof., Fife KY 16 9TY (GB); Frank, Timothy G., Dr., Wormit Newport-On-Tay, Fife KY169TY (GB); Brown, Stuart, Dr., St. Andrews, Fife KY 16 8QX (GB); Kelly, Leslie, Cupar, Fife KY15 5YL (GB); Rutherford, Ian, Dundee DD4 0RL (GB); Martin, J. Duncan S., Dundee DD2 2EZ (GB); Gove, James, Dundee DD4 9AJ (GB)
(74) Vertreter: Hofmeister, Frank

(56) Entgegenhaltungen:
- EP-A- 1 340 467
- WO-A-00/18306
- US-A- 5 634 937
- US-A1- 2001 049 502
- US-A1- 2003 055 437
- US-B1- 6 258 069
- US-B1- 6 551 270

## Beschreibung

Die Erfindung betrifft eine Vorrichtung zum Schaffen eines freien transkutanen Zuganges zu einem endoskopischen Operationsgebiet, insbesondere zu einem körperinneren Hohlorgan, mit einem in eine Inzision einsetzbaren konischen Einsatz.

Bei der minimal-invasiven Chirurgie werden transkutane Zugänge zum Operationsgebiet in der Regel mittels sogenannter Trokare geschaffen. Die Trokare bestehen aus einer steifen metallischen Hülse und dem in die Trokarhülse einschiebbaren Trokardorn, dem eigentlichen Trokar, der zum Ausbilden einer Inzision eine scharfe Spitze aufweist. Nach dem Ausbilden der Inzision wird die Trokarhülse in den Körper des Patienten eingeschoben und der Trokardorn wieder aus der Trokarhülse entfernt. Nachfolgend kann der Operateur durch die Trokarhülse chirurgische Instrumente, wie beispielsweise Endoskope und andere Operationsinstrumente, in den Körper einführen.

Bei inneren Hohlorganen, wie beispielsweise dem Magen, finden auch im anästhesierten Zustand in einem gewissen Umfang Relativbewegungen zwischen der Bauchdecke und dem inneren Hohlorgan statt, so dass hier relativ lange Trokarhülsen verwendet werden müssen, um sicherzustellen, dass die Trokarhülse aufgrund der Bewegungen des Hohlorgans nicht aus dem Hohlorgan herausrutscht.

Eine gattungsgemäße Vorrichtung zum Schaffen eines freien transkutanen Zuganges zu einem endoskopischen Operationsgebiet ist beispielsweise aus der US 5 634 937 A bekannt. Nachteilig an dieser Vorrichtung ist, dass über den konischen Einsatz nur jeweils ein medizinisches Instrument in das Operationsgebiet einführbar ist.

Aus der DE 199 16 088 A1 ist eine weitere Vorrichtung zum Schaffen eines transkutanen Zuganges zu einem körperinneren Hohlorgan bekannt. Diese bekannte für endoluminale Operationen dienende Vorrichtung besteht aus einem Fußteil, das klemmend an einer hohlen Sonde festlegbar ist, die vom Körperinneren her durch die Inzisionen in der Wand des Hohlorgans und der Bauchdecke nach außen geführt wird. An dieses solchermaßen an der Inzisionsstelle festgelegte Fußteil ist über ein Kupplungselement ein rohrförmiger Schaft anschließbar, der auf der Körperaußenseite die hohle Sonde so verlängert, dass medizinische Instrumente, wie beispielsweise Endoskope und andere Operationsinstrumente, über den hohlen Schaft und die Sonde in das Hohlorgan einführbar sind.

Diese bekannte Vorrichtung hat sich in der Praxis durchaus bewährt, jedoch ist einerseits der konstruktive Aufwand zur Herstellung der Vorrichtung relativ groß und sind andererseits das freie Lumen des hohle Schaftes sowie der Sonde nicht so groß bemessen, dass ein Endoskop und weitere Instrumente gleichzeitig und dann auch noch in dem üblichen Arbeitswinkel von 30° zueinander über diese Vorrichtung in das Operationsgebiet einführbar sind.

US-B-6551270 und US-A-2003/0055437 offenbaren Vorrichtungen gemäss dem Oberbegriff des ersten Anspruchs.

Davon ausgehend liegt der Erfindung die **Aufgabe** zugrunde, eine Vorrichtung zum Schaffen eines freien transkutanen Zuganges bereitzustellen, die bei einfachem Aufbau eine gute Handhabbarkeit für mehrere medizinische Instrumente bietet.

Die **Lösung** dieser Aufgabenstellung ist erfindungsgemäß dadurch gekennzeichnet, dass auf der Innenseite des konischen Einsatzes mehrere Führungsvorrichtungen für jeweils ein medizinisches Instrument ausgebildet sind, über die die einzelnen medizinischen Instrumente in einem festgelegten Winkel zueinander in dem konischen Einsatz angeordnet sind, wobei der durch die konische Innenfläche des Einsatzes aufgespannte Konuswinkel (d) 30° bis 90°, vorzugsweise 60°, betragt.

Die erfindungsgemäße Ausbildung der Vorrichtung ist es für den Operateur möglich, gleichzeitig mehrere Instrumente über einen konischen Einsatz in das Operationsgebiet einzuführen, wobei durch die festgelegte Winkelstellung der medizinischen Instrumente zueinander eine exakte Führung der Instrumente im Operationsgebiet gewährleistet wird.

Durch die Schaffung mehreren Führungsvorrichtungen, die erfindungsgemäß beispielsweise als in Konusrichtung des Einsatzes verlaufender Führungskanal ausgebildet sein können, kann der Operateur deutlich entlastet werden, da er das in dieser Führungsvorrichtung angeordnete Instrument, beispielsweise ein Endoskop, nicht mehr während der gesamten Operationsdauer mit einer Hand festhalten muss. Die Führungsvorrichtungen garantieren darüber hinaus eine fest vorgegebene Winkelstellung der medizinischen Instrumente zueinander.

Die bevorzugte Konuswinkel des erfindungsgemäßen Einsatzes sorgt dafür, dass der in der Praxis gewünschte Winkel von 30° zwischen der optischen Achse des Endoskops und den andere Instrumenten problemlos auch mit zwei Instrumenten einhaltbar ist.

Gemäß einer praktischen Ausführungsform der Erfindung wird vorgeschlagen, dass der konische Einsatz kegelstumpfförmig so ausgebildet ist, dass der Einsatz distalseitig eine im Durchmesser kleinere Öffnung aufweist als proximalseitig.

Um ein Herausrutschen des erfindungsgemäßen Einsatzes aus der Inzision zu verhindern, wird erfindungsgemäß vorgeschlagen, dass der konische Einsatz in der Inzision fixierbar ist. Hierzu wird gemäß einer bevorzugten Ausführungsform der Erfindung vorgeschlagen, dass auf der Außenseite des vorzugsweise aus einem formstabilen Kunststoffmaterial bestehenden Einsatzes mindestens ein Fixierelement angeordnet ist.

Gemäß einer ersten praktischen Ausführungsform der Erfindung wird vorgeschlagen, dass das mindestens eine Fixierelement als auf der Außenseite des Einsatzes angeordnete, zumindest eingängige Schraubenbahn ausgebildet ist, über die der Einsatz in die Inzision einschraubbar ist.

Gemäß einer zweiten erfindungsgemäßen Ausführungsform zur Ausbildung des mindestens einen Fixierelements wird vorgeschlagen, dass das mindestens eine Fixierelement als auf der Außenseite des Einsatzes angeordneter umlaufender Rastwulst ausgebildet ist. Vorteilhafterweise sind bei dieser Ausgestaltungsform auf der Außenseite des Einsatzes mindestens zwei parallel zueinander angeordnete umlaufende Rastwulste als Fixierelemente ausgebildet. Durch Reihen paralleler Rastwulste ist es möglich, die Einstecktiefe des Einsatzes in die Inzision zu variieren bzw. flexibel an die jeweiligen Operationsbedingungen anpassen zu können.

Um das Einfügen des konischen Einsatzes in die Inzision zu erleichtern und den in die Inzision eingefügten Einsatz relativ zu seiner Längsachse verkippen zu können, wird erfindungsgemäß vorgeschlagen, dass am Einsatz ein Führungsarm festlegbar ist. Zu diesem Zweck ist vorteilhafterweise auf der Außenseite des Einsatzes ein Kupplungselement zum Festlegen des Führungsarms angeordnet.

Schließlich wird mit der Erfindung vorgeschlagen, dass zumindest die proximalseitige Öffnung des Einsatzes über ein Abdeckelement verschließbar ist um das Operationsgebiet hygienisch gegen die Umgebung abzutrennen.

Gemäß einer ersten Ausführungsform der Erfindung ist das Abdeckelement als durch die in den Einsatz einzuführenden medizinischen Instrumente durchstoßbare Membran ausgebildet ist, wobei die Membran beispielsweise als Dichtungsscheibe aus einem Silikon-Material ausgebildet sein kann.

Mit einer zweiten Ausführungsform der Erfindung wird vorgeschlagen, dass das Abdeckelement als mit mindestens einer Öffnung zur Aufnahme eines in den konischen Einsatz einzuführenden medizinischen Instruments versehener Deckel ausgebildet ist, wobei jede Öffnung im Deckel mit einer elastischen Abdichtmembran versehen ist, welche den eingeführten Instrumentenschaft nach außen abdichten.

Um ein gezieltes Einführen der medizinischen Instrumente in das Operationsgebiet zu erleichtern, kann der Deckel erfindungsgemäß derart verdrehsicher am konischen Einsatz festlegbar ist, dass im verschlossenen Zustand zumindest eine Öffnung im Deckel mit einer auf der Innenseite des konischen Einsatzes angeordneten Führungsvorrichtung fluchtet. Ein in die Deckelöffnung eingesetztes medizinisches Instrument wird dann über die Führungsvorrichtung positionsgenau dem Operationsgebiet zugeführt. Vorteilhafterweise besteht der Deckel aus einem transparenten Material, insbesondere einem Kunststoffmaterial, damit durch den transparenten Deckel festgestellt werden kann, ob die eingesetzten medizinischen Instrumente Aufnahme in den Führungsvorrichtungen auf der Innenseite des konischen Einsatzes gefunden haben.

Die Abdichtung des Deckels gegenüber dem konischen Einsatz erfolgt erfindungsgemäß über ein zwischengelagertes Dichtungselement.

Weitere Merkmale und Vorteile der Erfindung ergeben sich anhand der zugehörigen Zeichnung, in der ein Ausführungsbeispiel einer erfindungsgemäßen Vorrichtung zum Schaffen eines freien transkutanen Zuganges zu einem endoskopischen Operationsgebiet nur beispielhaft dargestellt ist. In der Zeichnung zeigt:
- Fig. 1: eine schematische Darstellung einer Operation mit einer erfindungsgemäßen Vorrichtung;
- Fig. 2: eine perspektivische Seitenansicht einer erfindungsgemäßen Vorrichtung;
- Fig. 3: eine Ansicht von oben auf die Vorrichtung gemäß Fig. 2 und
- Fig. 4: eine schematische Schnittdarstellung, eine erfindungsgemäße Vorrichtung im in die Bauchdecke und die Magenwand eingesetzten Zustand darstellend.

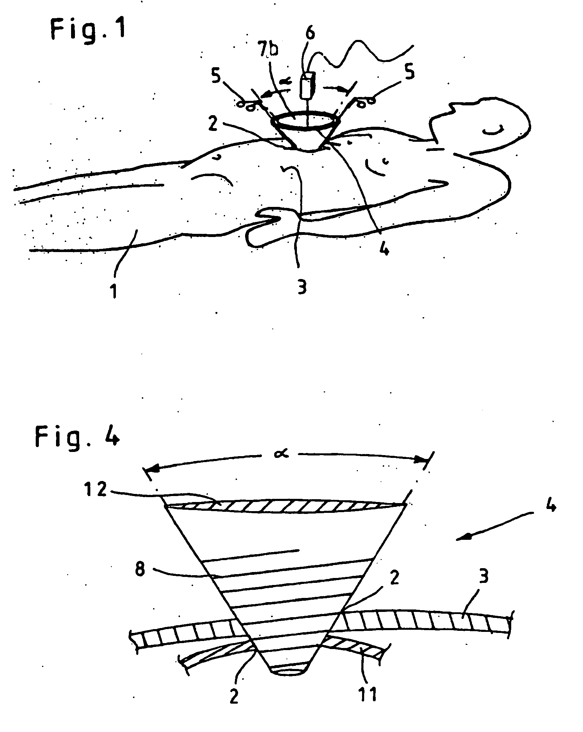

In Abbildung Fig. 1 ist beispielhaft schematisch eine endoskopische Operation eines Patienten 1 dargestellt. Zu Beginn der Operation wird beispielsweise mittels eines Trokardorns eine Inzision 2 in der Bauchdecke 3 des Patienten 1 ausgebildet. In diese Inzision 2 wird nachfolgend ein konischer Einsatz 4 eingesetzt, der einen freien transkutanen Zugang zum Einführen von medizinischen Instrumenten, wie beispielsweise Schneid- und/oder Greifinstrumenten 5 und Endoskopen oder Kameras 6, in das Operationsgebiet ermöglicht.

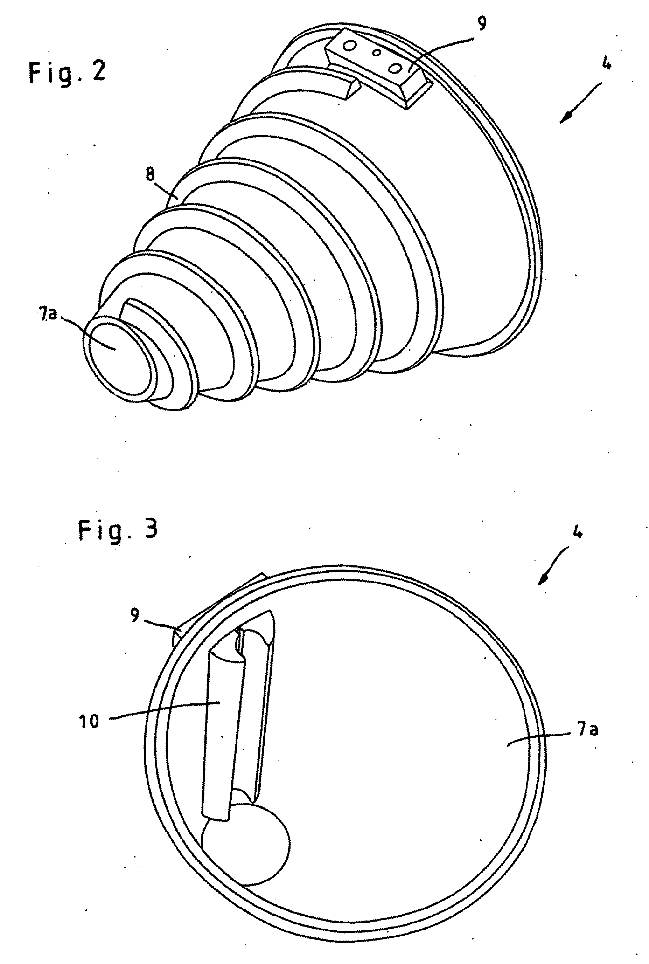

Die konstruktive Ausgestaltung des konischen Einsatzes 4 ist genauer den Darstellungen gemäß Fig. 2 und 3 zu entnehmen. Wie insbesondere aus Fig. 2 ersichtlich, ist der vorzugsweise aus einem formstabilen Kunststoffmaterial bestehende konische Einsatz 4 bei der dargestellten Ausführungsform kegelstumpfförmig so ausgebildet, dass der Einsatz 4 distalseitig eine im Durchmesser kleinere Öffnung 7a aufweist als proximalseitig.

Der durch die konische Innenfläche des Einsatzes 4 aufgespannte Konuswinkel α beträgt vorzugsweise 60°. Dieser Winkel ist besonders vorteilhaft, da bei dieser Ausgestaltung, wie aus Fig. 1 ersichtlich, mindestens zwei Schneid- und/oder Greifinstrumente 5 sowie ein Endoskop oder eine Kamera 6 so über den konischen Einsatz 4 in das Operationsgebiet einführbar sind, dass der Winkel zwischen der optischen Achse des Endoskops bzw. der Kamera 6 und der Längsachse der Schneid- und/oder Greifinstrumente 5 die in Praxis gewünschten 30° einnehmen kann. Diese Winkelstellung ermöglicht dem Operateur eine Stellung des optischen Werkzeugs und der eigentlichen Arbeitswerkzeuge zueinander, wie dies ansonsten nur unter Verwendung von mindestens zwei Inzisionen 2 möglich ist.

Um den konischen Einsatz 4 in der Inzision 2 fixieren zu können und, um so ein Herausrutschen des Einsatzes 4 aus der Inzision 2 zu verhindern, ist bei der dargestellten Ausführungsform auf der Außenseite des Einsatzes 4 ein als Schraubenbahn 8 ausgebildetes Fixierelement angeordnet. Über diese Schraubenbahn 8, die auch aus mehreren parallelen Schraubenbahnen 8 bestehen kann, lässt sich der konische Einsatz 4 einfach und schnell in die Inzision 2 einschrauben, wie dies in Fig. 4 dargestellt ist.

Alternativ zu der dargestellten Ausführungsform ist es beispielsweise auch möglich, das Fixierelement als auf der Außenseite des Einsatzes 4 angeordneter umlaufender Rastwulst auszubilden, wobei es bei dieser Ausgestaltungsform vorteilhaft ist, mehrere parallel zueinander angeordnete Rastwulste zu verwenden, um die Einstecktiefe des Einsatzes 4 in die Inzision 2 zu variieren bzw. flexibel an die jeweiligen Operationsbedingungen anpassen zu können.

Das Einstecken bzw. Einschrauben des konischen Einsatzes 4 in die Inzision 2 kann dadurch erleichtert werden, dass am Einsatz 4 ein radial nach außen abstehender Führungsarm festlegbar ist. Bei der in Fig. 2 dargestellten Ausführungsform ist zwar nicht der Führungsarm an sich dargestellt, jedoch ein auf der Außenseite des Einsatzes 4 angeordnetes Kupplungselement 9 zum Festlegen des Führungsarms. Der Führungsarm kann weiterhin dafür verwendet werden, den in der Operationsposition in der Inzision 2 befindlichen Einsatz 4 im Ganzen relativ zur Bauchdecke 3 des Patienten 1 zu verkippen.

Die Draufsicht auf den konischen Einsatz 4 gemäß Fig. 3 ist zu entnehmen, dass bei der dargestellten Ausführungsform auf der Innenseite des Einsatzes 4 eine als in Konusrichtung des Einsatzes 4 verlaufender Führungskanal ausgebildete Führungsvorrichtung 10 angeordnet ist. Durch die Schaffung dieser Führungsvorrichtung 10 kann der Operateur entlastet werden, da er das in dieser Führungsvorrichtung 10 angeordnete Instrument, beispielsweise ein Endoskop 6, nicht mehr während der gesamten Operationsdauer mit einer Hand festhalten muss.

Die Abbildung Fig. 4 zeigt schließlich die Verwendung eines wie zuvor beschriebenen konischen Einsatzes 4 bei einer endoluminalen Operation an einem köperinneren Hohlorgan, in diesem Fall dem Magen. Bei dieser Operation wird der konische Einsatz 4 in Inzisionen 2 in der Bauchdecke 3 sowie der Magenwand 11 des Patienten 1 eingesetzt, so dass dem Operateur ein direkter und einfach anzulegender transkutaner Zugang zum Operationsgebiet zur Verfügung steht, der dem Operateur einen großen Bewegungsspielraum zur Handhabung der medizinischen Operationsinstrumente ermöglicht. Die Schnittdarstellung gemäß Fig. 4 zeigt deutlich, wie die als Fixierelement dienende Schraubenbahn 8 auf der Außenseite des konischen Einsatzes 4 die einzelnen Gewebepartien, nämlich die Bauchdecke 3 einerseits und die Magenwand 11 andererseits, hintergreift und so ein versehentliches Herausrutschen des konischen Einsatzes 4 aus den Inzisionen 2 verhindert.

Wie weiterhin aus Fig. 4 ersichtlich, ist zumindest die proximalseitige Öffnung 7b des konischen Einsatzes 4 über ein Abdeckelement 12 verschließbar, um das Operationsgebiet hygienisch gegenüber der Umgebung abzudichten. Bei der dargestellten Ausführungsform ist das Abdeckelement 12 als Membran ausgebildet, die aus einem elastischen und von den in den konischen Einsatz 4 einzuführenden Instrumenten durchstoßbaren Material besteht, so dass sich die Membran auch bei eingesetzten Operationsinstrumenten abdichtend an die jeweiligen Instrumentenschäfte anlegt. Als Material zur Ausbildung der selbstschließenden und selbstdichtenden Abdeckmembran ist beispielsweise ein Silikon-Material geeignet.

Alternativ zu der dargestellten Ausbildung des Abdeckelements 12 als Membran ist es selbstverständlich auch möglich, das Abdeckelement 12 als am konischen Einsatz 4 festlegbarer Deckel auszubilden, in dem zur Aufnahme eines in den konischen Einsatz einzuführenden medizinischen Instruments mindestens eine Öffnung ausgebildet ist und wobei jede Öffnung im Deckel mit einer elastischen Abdichtmembran versehen ist, welche den eingeführten Instrumentenschaft nach außen abdichtet.

Um ein gezieltes Einführen der medizinischen Instrumente in das Operationsgebiet zu erleichtern, ist der Deckel verdrehsicher so am konischen Einsatz 4 festlegbar, dass im verschlossenen Zustand zumindest eine Öffnung im Deckel mit mehrere auf der Innenseite des konischen Einsatzes 4 angeordneten Führungsvorrichtungen 10 fluchtet. Ein in die Deckelöffnung eingesetztes medizinisches Instrument wird dann über die Führungsvorrichtung 10 positionsgenau dem Operationsgebiet zugeführt. Durch den aus einem transparenten Material, insbesondere einem Kunststoffmaterial, bestehenden Deckel kann so von außen festgestellt werden kann, ob die eingesetzten medizinischen Instrumente Aufnahme in den Führungsvorrichtungen 10 auf der Innenseite des konischen Einsatzes 4 gefunden haben.

Die Handhabung der zuvor beschriebenen Vorrichtung zum Schaffen eines transkutanen Zuganges zu einem endoskopischen Operationsgebiet geschieht wie folgt:

Zu Beginn der Operation wird mittels eines spitzen Trokardorns eine Inzision 2 in der Bauchdecke 3 des Patienten 1 ausgebildet. Bei einer endoluminalen Operation wird mittels des Trokardorns eine zweite Inzision 2 in der Außenwand des körperinneren Hohlorgans ausgebildet. Bei einer endoluminalen Magenoperation erfolgt die Ausbildung der Inzision 2 in der Magenwand vorteilhafterweise unter Kontrolle und Führung eines über die Speiseröhre in den Magen eingeführten Gastroskops.

Anschließend an die Ausbildung der Inzision 2 bzw. der Inzisionen 2 wird auf den Trokardorn als Führung der konische Einsatz 4 aufgesetzt und mittels der Schraubenbahn 8 in die Inzision/Inzisionen 8 eingeschraubt. Die als Fixierelement dienende Schraubenbahn 8 hält den konischen Einsatz 4 sicher und positionsgenau im Körper des Patienten 1, so dass der Trokardorn nachfolgend wieder aus der Inzision / den Inzisionen 2 herausgezogen werden kann.

Nun steht dem Operateur ein leicht zugänglicher transkutaner Zugang zum Operationsgebiet zur Verfügung. Aufgrund des großen Konuswinkels α des konischen Einsatzes 4 können mehrere Operationsinstrumente gleichzeitig über den nur einen transkutanen Zugang in das Operationsgebiet eingeführt werden, wobei auch der vorteilhafte Anstellwinkel der Arbeitsinstrumente zur optischen Achse eines Sichtinstruments von 30° eingehalten werden kann. Die gute Zugänglichkeit des Operationsgebiets erleichtert es darüber hinaus abgetrenntes Gewebe aus dem Körper zu entfernen und/oder beispielsweise Nahtmaterial in den Körper einzuführen.

Nach erfolgter Operation wird der Einsatz 4 wieder aus der Inzision 2 herausgeschraubt und die Inzisionsstelle vernäht. Bei der in Fig. 4 dargestellten endoluminalen Operation wird der konische Einsatz 4 zunächst nur aus der Inzision 2 in der Magenwand 11 herausgeschraubt, um nachfolgend die Inzision 2 in der Magenwand 11 durch eine Naht zu verschließen.

Eine wie zuvor beschrieben ausgebildete Vorrichtung zum Schaffen eines transkutanen Zuganges zu einem endoskopischen Operationsgebiet zeichnet sich dadurch aus, dass sie bei einem besonders einfachen konstruktiven Aufbau dem Operateur eine größtmögliche Bewegungsfreiheit bei der Operation ermöglicht.

Für den Patienten 1 ist die Verwendung des konischen Einsatzes 4 vorteilhaft, da in der Regel auf die Ausbildung einer zweiten oder gar dritten Inzision 2 zum Zuführen weiterer Operationsinstrumente verzichtet werden kann.

### Bezugszeichenliste

- 1: Patient
- 2: Inzision
- 3: Bauchdecke
- 4: Einsatz
- 5: Schneid- / Greifinstrument
- 6: Endoskop / Kamera
- 7a: distale Öffnung
- 7b: proximale Öffnung
- 8: Schraubenbahn
- 9: Kupplungselement
- 10: Führungsvorrichtung
- 11: Magenwand
- 12: Abdeckelement

- α: Konuswinkel

## Patentansprüche

1. Vorrichtung zum Schaffen eines freien transkutanen Zuganges zu einem endoskopischen Operationsgebiet, insbesondere zu einem körperinneren Hohlorgan, mit einem in eine Inzision (2) einsetzbaren konischen Einsatz (4), wobei mehrere medizinische Instrument gleichzeitig über den konischen Einsatz (4) in das Operationsgebiet einführbar sind,
**dadurch gekennzeichnet,**
**dass** auf der Innenseite des konischen Einsatzes (4) mehrere Führungsvorrichtungen (10) für jeweils ein medizinisches Instrument ausgebildet sind, über die die einzelnen medizinischen Instrumente in einem festgelegten Winkel zueinander in dem konischen Einsatz (4) angeordnet sind, wobei der durch die konische Innenfläche des Einsatzes (4) aufgespannte Konuswinkel (α) 30° bis 90°, vorzugsweise 60°, beträgt.

2. Vorrichtung nach Anspruch 1, **dadurch gekennzeichnet, dass** die Führungsvorrichtung (10) als in Konusrichtung des Einsatzes (4) verlaufender Führungskanal ausgebildet ist.

3. Vorrichtung nach Anspruch 1 oder 2, **dadurch gekennzeichnet, dass** der konische Einsatz (4) kegelstumpfförmig so ausgebildet ist, dass der Einsatz (4) distalseitig eine im Durchmesser kleinere Öffnung (7a) aufweist als proximalseitig

4. Vorrichtung nach einem der Ansprüche 1 bis 3, **dadurch gekennzeichnet, dass** der konische Einsatz (4) aus einem im Wesentlichen formstabilen Kunststoffmaterial besteht.

5. Vorrichtung nach einem der Ansprüche 1 bis 4, **dadurch gekennzeichnet, dass** der konische Einsatz (4) in der mindestens einen Inzision (2) fixierbar ist..

6. Vorrichtung nach Anspruch 5, **dadurch gekennzeichnet, dass** auf der Außenseite des konischen Einsatzes (4) mindestens ein Fixierelement angeordnet ist.

7. Vorrichtung nach Anspruch 6, **dadurch gekennzeichnet, dass** das mindestens eine Fixierelement als auf der Außenseite des konischen Einsatzes (4) angeordnete, zumindest eingängige Schraubenbahn (8) ausgebildet ist.

8. Vorrichtung nach Anspruch 6, **dadurch gekennzeichnet, dass** das mindestens eine Fixierelement als auf der Außenseite des konischen Einsatzes (4) angeordneter umlaufender Rastwulst ausgebildet ist.

9. Vorrichtung nach Anspruch 8, **dadurch gekennzeichnet, dass** auf der Außenseite des konischen Einsatzes (4) mindestens zwei parallel zueinander angeordnete umlaufende Rastwulste als Fixierelemente ausgebildet sind.

10. Vorrichtung nach einem der Ansprüche 1 bis 9, **dadurch gekennzeichnet, dass** am konischen Einsatz (4) ein Führungsarm festlegbar ist.

11. Vorrichtung nach Anspruch 10, **dadurch gekennzeichnet, dass** auf der Außenseite des konischen Einsatzes (4) ein Kupplungselement (9) zum Festlegen des Führungsarms angeordnet ist.

12. Vorrichtung nach einem der Ansprüche 1 bis 11, **dadurch gekennzeichnet, dass** zumindest die proximalseitige Öffnung (7b) des konischen Einsatzes (4) über ein Abdeckelement (12) verschließbar ist.

13. Vorrichtung nach Anspruch 12, **dadurch gekennzeichnet, dass** das Abdeckelement (12) als Membran ausgebildet ist.

14. Vorrichtung nach Anspruch 13, **dadurch gekennzeichnet, dass** die Membran als durch die in den konischen Einsatz (4) einzuführenden medizinischen Instrumente durchstoßbare Membran (12), beilspielsweise aus einem SilikonMaterial, ausgebildet ist.

15. Vorrichtung nach Anspruch 12, **dadurch gekennzeichnet, dass** das Abdeckelement (12) als mit mindestens einer Öffnung zur Aufnahme eines in den konischen Einsatz (4) einzuführenden medizinischen Instruments versehener Deckel ausgebildet ist, wobei jede Öffnung im Deckel mit einer elastischen Abdichtmembran versehen ist.

16. Vorrichtung nach Anspruch 15, **dadurch gekennzeichnet, dass** der Deckel derart verdrehsicher am konischen Einsatz (4) festlegbar ist, dass im verschlossenen Zustand zumindest eine Öffnung im Deckel mit einer auf der Innenseite des konischen Einsatzes (4) angeordneten Führungsvorrichtung (10) fluchtet.

17. Vorrichtung nach einem der Ansprüche 14 bis 16 **dadurch gekennzeichnet, dass** der Deckel aus einem transparenten Material, insbesondere einem Kunststoffmaterial, besteht.

18. Vorrichtung nach einem der Ansprüche 14 bis 17 **dadurch gekennzeichnet, dass** der Deckel gegenüber dem konischen Einsatz (4) über ein Dichtungselement abgedichtet ist.

## Claims

1. Device for establishing free transcutaneous access to an endoscopic surgical area, in particular to a hollow organ in the body, with a conical insert (4) that can be inserted into an incision (2), such that several medical instruments can be guided simultaneously via the conical insert (4) into the surgical area, **characterized in that** the inner face of the conical insert (4) has several guide means (10) which are provided in each case for a medical instrument and via which the individual medical instruments are arranged at a fixed angle to one another in the conical insert (4), the cone angle (α) spanned by the conical inner face of the insert (4) being from 30° to 90°, preferably 60°.

2. Device according to Claim 1, **characterized in that** the guide means (10) is designed as a guide channel extending in the cone direction of the insert (4).

3. Device according to Claim 1 or 2, **characterized in that** the conical insert (4) has a frustoconical design such that the distal end of the insert (4) has an opening (7a) of smaller diameter than the proximal end.

4. Device according to one of Claims 1 to 3, **characterized in that** the conical insert (4) is made of a substantially dimensionally stable plastic material.

5. Device according to one of Claims 1 to 4, **characterized in that** the conical insert (4) can be fixed in the at least one incision (2).

6. Device according to Claim 5, **characterized in that** at least one fixing element is arranged on the outer face of the conical insert (4).

7. Device according to Claim 6, **characterized in that** the at least one fixing element is designed as an at least single helical path (8) arranged on the outer face of the conical insert (4).

8. Device according to Claim 6, **characterized in that** the at least one fixing element is designed as a circumferential locking bead arranged on the outer face of the conical insert (4).

9. Device according to Claim 8, **characterized in that** at least two parallel circumferential locking beads are formed as fixing elements on the outer face of the conical insert (4).

10. Device according to one of Claims 1 to 9, **characterized in that** a guide arm can be secured on the conical insert (4).

11. Device according to Claim 10, **characterized in that** a coupling element (9) for securing the guide arm is arranged on the outer face of the conical insert (4).

12. Device according to one of Claims 1 to 11, **characterized in that** at least the proximal opening (7b) of the conical insert (4) can be closed by a cover element (12).

13. Device according to Claim 12, **characterized in that** the cover element (12) is designed as a membrane.

14. Device according to Claim 13, **characterized in that** the membrane is designed as a membrane (12), for example of a silicone material, that can be pierced by the medical instruments to be introduced into the conical insert (4).

15. Device according to Claim 12, **characterized in that** the cover element (12) is designed as a lid provided with at least one opening for receiving a medical instrument to be introduced into the conical insert (4), each opening in the lid being provided with an elastic sealing membrane.

16. Device according to Claim 15, **characterized in that** the lid can be secured against twisting on the conical insert (4) in such a way that, in the closed state, at least one opening in the lid is aligned with a guide means (10) arranged on the inner face of the conical insert (4).

17. Device according to one of Claims 14 to 16, **characterized in that** the lid is made of a transparent material, in particular a plastic material.

18. Device according to one of Claims 14 to 17, **characterized in that** the lid is sealed with respect to the conical insert (4) by a sealing element.

## Revendications

1. Dispositif permettant de pratiquer un accès transcutané libre à une zone d'opération chirurgicale endoscopique, en particulier à un organe creux interne du corps, comprenant un insert (4) conique qui peut être engagé dans une incision (2), plusieurs instruments médicaux pouvant être introduits simultanément dans la zone d'opération chirurgicale par l'intermédiaire de l'insert (4) conique,
**caractérisé**
**en ce que** sur le côté intérieur de l'insert (4) conique sont formés plusieurs dispositifs de guidage (10) destinés chacun à un instrument médical, et par l'intermédiaire desquels les différents instruments médicaux individuels sont agencés dans l'insert (4) conique selon un angle déterminé les uns par rapport aux autres, l'angle de cône (α) défini par la surface intérieure conique de l'insert (4) valant de 30° à 90°, de préférence 60°.

2. Dispositif selon la revendication 1, **caractérisé en ce que** le dispositif de guidage (10) est réalisé sous la forme d'un canal de guidage s'étendant dans la direction de cône de l'insert (4).

3. Dispositif selon la revendication 1 ou 2, **caractérisé en ce que** l'insert (4) conique est réalisé sous forme de tronc de cône de manière telle que l'insert (4) présente, côté distal, une ouverture (7a) plus petite en diamètre que côté proximal.

4. Dispositif selon l'une des revendications 1 à 3, **caractérisé en ce que** l'insert (4) conique est réalisé en une matière plastique de forme sensiblement stable.

5. Dispositif selon l'une des revendications 1 à 4, **caractérisé en ce que** l'insert (4) conique peut être fixé dans ladite une incision (2).

6. Dispositif selon la revendication 5, **caractérisé en ce que** sur le côté extérieur de l'insert (4) conique est agencé au moins un élément de fixation.

7. Dispositif selon la revendication 6, **caractérisé en ce que** ledit élément de fixation est réalisé en tant que voie en hélice (8) à au moins un filet, agencée sur le côté extérieur de l'insert (4) conique.

8. Dispositif selon la revendication 6, **caractérisé en ce que** ledit élément de fixation est réalisé en tant que bourrelet d'encliquetage périphérique, agencé sur le côté extérieur de l'insert (4) conique.

9. Dispositif selon la revendication 8, **caractérisé en ce que** sur le côté extérieur de l'insert (4) conique sont formés au moins deux bourrelets d'encliquetage périphériques agencés parallèlement l'un à l'autre, en tant qu'éléments de fixation.

10. Dispositif selon l'une des revendications 1 à 9, **caractérisé en ce que** sur l'insert (4) conique peut être fixé un bras de guidage.

11. Dispositif selon la revendication 10, **caractérisé en ce que** sur le côté extérieur de l'insert (4) conique est agencé un élément d'accouplement (9) pour la fixation du bras de guidage.

12. Dispositif selon l'une des revendications 1 à 11, **caractérisé en ce qu'**au moins l'ouverture proximale (7b) de l'insert (4) conique peut être fermée par un élément de recouvrement (12).

13. Dispositif selon la revendication 12, **caractérisé en ce que** l'élément de recouvrement (12) est réalisé sous la forme d'une membrane.

14. Dispositif selon la revendication 13, **caractérisé en ce que** la membrane est réalisée sous forme de membrane (12) pouvant être transpercée par les instruments médicaux à introduire dans l'insert (4) conique, par exemple en un matériau du type silicone.

15. Dispositif selon la revendication 12, **caractérisé en ce que** l'élément de recouvrement (12) est réalisé sous la forme d'un couvercle muni d'au moins une ouverture destinée à recevoir un instrument médical à introduire dans l'insert (4) conique, chaque ouverture dans le couvercle étant pourvue d'une membrane élastique d'étanchéité.

16. Dispositif selon la revendication 15, **caractérisé en ce que** le couvercle peut être fixé ou immobilisé de manière bloquée en rotation sur l'insert (4) conique, de façon telle que dans l'état fermé, au moins une ouverture dans le couvercle soit alignée avec un dispositif de guidage (10) agencé sur le côté intérieur de l'insert (4) conique.

17. Dispositif selon l'une des revendications 14 à 16, **caractérisé en ce que** le couvercle est réalisé en un matériau transparent, notamment une matière plastique.

18. Dispositif selon l'une des revendications 14 à 17, **caractérisé en ce que** le couvercle est rendu étanche par rapport à l'insert (4) conique par l'intermédiaire d'un élément de joint d'étanchéité.
